# FASCICULE DE BREVET EUROPEEN

(11) **EP 0 848 720 B1**
(45) Date de publication et mention de la délivrance du brevet: **14.01.2004**
(21) Numéro de dépôt: 96930195.1
(22) Date de dépôt: 02.09.1996
(51) Int. Cl.: A61K 38/16, A61K 48/00, A61K 39/39, C12N 15/86, A61P 35/00

(54) **ANTAGONISTES DE L'ACTIVITE ONCOGENIQUE DE LA PROTEINE MDM2, ET LEUR UTILISATION DANS LE TRAITEMENT DES CANCERS**
ANTAGONISTEN DER ONKOGENEN AKTIVITÄT VON MDM2, UND IHRE VERWENDUNG IN DER KREBSBEHANDLUNG
ANTAGONISTS OF THE ONCOGENIC ACTIVITY OF THE PROTEIN MDM2, AND USE THEREOF IN THE TREATMENT OF CANCERS

(30) Priorité: 04.09.1995 FR 9510331
(43) Date de publication de la demande: 24.06.1998
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: TOCQUE, Bruno, F-94200 Courbevoie (FR); DUBS-POTERSZMAN, Marie-Christine, F-67202 Wolfisheim (FR); WASYLYK, Bohdan, F-67400 Illkirsch (FR)
(74) Mandataire: Savina, Jacques
(86) Numéro de dépôt international: PCT/FR1996/001340
(87) Numéro de publication internationale: WO 1997/009343

(56) Documents cités:
- WO-A-93/20238
- WO-A-94/29446
- WO-A-96/02642
- INTERNATIONAL JOURNAL OF ONCOLOGY, vol. 6, no. 1, Janvier 1995, pages 251-259, XP002004851 PING LENG ET AL.: "HUMAN ONCOPROTEIN MDM2 INTERACTS WITH THE TATA-BINDING PROTEIN IN VITRO AND IN VIVO"
- JOURNAL OF CELLULAR BIOCHEMISTRY, no. SUPPLEMENT 19A, 5 - 26 Janvier 1995, page 54 XP000572208 THIERRY L VEILLARD ET AL.: "THE ONCOPROTEIN MDM2 ASSOCIATES WITH THE TATA-BINDING PROTEIN (TBP)."
- MOLECULAR AND CELLULAR BIOLOGY, vol. 13, no. 7, Juillet 1993, pages 4107-4114, XP000571629 JIANDONG CHEN ET AL.: "MAPPING OF THE P53 AND MDM-2 INTERACTION DOMAINS."

### LISTE DE SEQUENCES

(1) INFORMATIONS GENERALES:
   (i) DEPOSANT:
      (A) NOM: RHONE POULENC RORER S.A.
      (B) RUE: 20, Avenue Raymond Aron
      (C) VILLE: ANTONY
      (E) PAYS: FRANCE
      (F) CODE POSTAL: 92165
      (G) TELEPHONE: 40.91.69.22
      (H) TELECOPIE: (1) 40.91.72.96
   (ii) DEPOSANT:
      (A) NOM: INSTITUT NATIONAL DE LA SANTE ET DE LA RECHERCHE MEDICALE (INSERM)
      (B) RUE: 101, Rue de Tolbiac
      (C) VILLE: PARIS Cedex 13
      (E) PAYS: FRANCE
      (F) CODE POSTAL:75654
      (G) TELEPHONE: 44.23.60.61.
      (H) TELECOPIE: 45.85.68.56.
   (ii) TITRE DE L' INVENTION: ANTAGONISTES DE L'ACTIVITE ONCOGENIQUE DE LA PROTEINE MDM2 ET LEUR UTILISATION DANS LE TRAITEMENT DES CANCERS
   (iii) NOMBRE DE SEQUENCES: 2
   (iv) FORME DECHIFFRABLE PAR ORDINATEUR:
      (A) TYPE DE SUPPORT: Tape
      (B) ORDINATEUR: IBM PC compatible
      (C) SYSTEME D' EXPLOITATION: PC-DOS/MS-DOS
      (D) LOGICIEL: PatentIn Release #1.0, Version #1.30 (OEB)
(2) INFORMATIONS POUR LA SEQ ID NO: 1:
   (i) CARACTERISTIQUES DE LA SEQUENCE:
      (A) LONGUEUR: 1476 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1476
   (xi) DESCRIPTION DE LA SEQUENCE: SEQ ID NO: 1:
   ATG TGC AAT ACC AAC ATG TCT GTA CCT ACT GAT GGT GCT GTA ACC ACC      48
   Met Cys Asn Thr Asn Met Ser Val Pro Thr Asp Gly Ala Val Thr Thr
    1               5                  10                  15
   TCA CAG ATT CCA GCT TCG GAA CAA GAG ACC CTG GTT AGA CCA AAG CCA      96
   Ser Gln Ile Pro Ala Ser Glu Gln Glu Thr Leu Val Arg Pro Lys Pro
               20                  25                  30
   TTG CTT TTG AAG TTA TTA AAG TCT GTT GGT GCA CAA AAA GAC ACT TAT     144
   Leu Leu Leu Lys Leu Leu Lys Ser Val Gly Ala Gln Lys Asp Thr Tyr
           35                  40                  45
   ACT ATG AAA GAG GTT CTT TTT TAT CTT GGC CAG TAT ATT ATG ACT AAA     192
   Thr Met Lys Glu Val Leu Phe Tyr Leu Gly Gln Tyr Ile Met Thr Lys
        50                  55                  60
   CGA TTA TAT GAT GAG AAG CAA CAA CAT ATT GTA TAT TGT TCA AAT GAT     240
   Arg Leu Tyr Asp Glu Lys Gln Gln His Ile Val Tyr Cys Ser Asn Asp
    65                  70                  75                  80
   CTT CTA GGA GAT TTG TTT GGC GTG CCA AGC TTC TCT GTG AAA GAG CAC     288
   Leu Leu Gly Asp Leu Phe Gly Val Pro Ser Phe Ser Val Lys Glu His
                    85                  90                  95
   AGG AAA ATA TAT ACC ATG ATC TAC AGG AAC TTG GTA GTA GTC AAT CAG     336
   Arg Lys Ile Tyr Thr Met Ile Tyr Arg Asn Leu Val Val Val Asn Gln
               100                 105                 110
   CAG GAA TCA TCG GAC TCA GGT ACA TCT GTG AGT GAG AAC AGG TGT CAC     384
   Gln Glu Ser Ser Asp Ser Gly Thr Ser Val Ser Glu Asn Arg Cys His
           115                 120                 125
   CTT GAA GGT GGG AGT GAT CAA AAG GAC CTT GTA CAA GAG CTT CAG GAA     432
   Leu Glu Gly Gly Ser Asp Gln Lys Asp Leu Val Gln Glu Leu Gln Glu
       130                 135                 140
   GAG AAA CCT TCA TCT TCA CAT TTG GTT TCT AGA CCA TCT ACC TCA TCT     480
   Glu Lys Pro Ser Ser Ser His Leu Val Ser Arg Pro Ser Thr Ser Ser
   145                 150                 155                 160
   AGA AGG AGA GCA ATT AGT GAG ACA GAA GAA AAT TCA GAT GAA TTA TCT     528
   Arg Arg Arg Ala Ile Ser Glu Thr Glu Glu Asn Ser Asp Glu Leu Ser
   165 170 175
   GGT GAA CGA CAA AGA AAA CGC CAC AAA TCT GAT AGT ATT TCC CTT TCC 576
   Gly Glu Arg Gln Arg Lys Arg His Lys Ser Asp Ser Ile Ser Leu Ser
   180 185 190
   TTT GAT GAA AGC CTG GCT CTG TGT GTA ATA AGG GAG ATA TGT TGT GAA 624
   Phe Asp Glu Ser Leu Ala Leu Cys Val Ile Arg Glu Ile Cys Cys Glu
   195 200 205
   AGA AGC AGT AGC AGT GAA TCT ACA GGG ACG CCA TCG AAT CCG GAT CTT 672
   Arg Ser Ser Ser Ser Glu Ser Thr Gly Thr Pro Ser Asn Pro Asp Leu
   210 215 220
   GAT GCT GGT GTA AGT GAA CAT TCA GGT GAT TGG TTG GAT CAG GAT TCA 720
   Asp Ala Gly Val Ser Glu His Ser Gly Asp Trp Leu Asp Gln Asp Ser
   225 230 235 240
   GTT TCA GAT CAG TTT AGT GTA GAA TTT GAA GTT GAA TCT CTC GAC TCA 768
   Val Ser Asp Gln Phe Ser Val Glu Phe Glu Val Glu Ser Leu Asp Ser
   245 250 255
   GAA GAT TAT AGC CTT AGT GAA GAA GGA CAA GAA CTC TCA GAT GAA GAT 816
   Glu Asp Tyr Ser Leu Ser Glu Glu Gly Gln Glu Leu Ser Asp Glu Asp
   260 265 270
   GAT GAG GTA TAT CAA GTT ACT GTG TAT CAG GCA GGG GAG AGT GAT ACA 864
   Asp Glu Val Tyr Gln Val Thr Val Tyr Gln Ala Gly Glu Ser Asp Thr
           275                 280                 285
   GAT TCA TTT GAA GAA GAT CCT GAA ATT TCC TTA GCT GAC TAT TGG AAA    912
   Asp Ser Phe Glu Glu Asp Pro Glu Ile Ser Leu Ala Asp Tyr Trp Lys
       290                 295                 300
   TGC ACT TCA TGC AAT GAA ATG AAT CCC CCC CTT CCA TCA CAT TGC AAC    960
   Cys Thr Ser Cys Asn Glu Met Asn Pro Pro Leu Pro Ser His Cys Asn
   305                 310                 315                 320
   AGA TGT TGG GCC CTT CGT GAG AAT TGG CTT CCT GAA GAT AAA GGG AAA   1008
   Arg Cys Trp Ala Leu Arg Glu Asn Trp Leu Pro Glu Asp Lys Gly Lys
                   325                 330                 335
   GAT AAA GGG GAA ATC TCT GAG AAA GCC AAA CTG GAA AAC TCA ACA CAA   1056
   Asp Lys Gly Glu Ile Ser Glu Lys Ala Lys Leu Glu Asn Ser Thr Gln
               340                 345                 350
   GCT GAA GAG GGC TTT GAT GTT CCT GAT TGT AAA AAA ACT ATA GTG AAT   1104
   Ala Glu Glu Gly Phe Asp Val Pro Asp Cys Lys Lys Thr Ile Val Asn
           355                 360                 365
   GAT TCC AGA GAG TCA TGT GTT GAG GAA AAT GAT GAT AAA ATT ACA CAA   1152
   Asp Ser Arg Glu Ser Cys Val Glu Glu Asn Asp Asp Lys Ile Thr Gln
       370                 375                 380
   GCT TCA CAA TCA CAA GAA AGT GAA GAC TAT TCT CAG CCA TCA ACT TCT   1200
   Ala Ser Gln Ser Gln Glu Ser Glu Asp Tyr Ser Gln Pro Ser Thr Ser
   385                 390                 395                 400
   AGT AGC ATT ATT TAT AGC AGC CAA GAA GAT GTG AAA GAG TTT GAA AGG   1248
   Ser Ser Ile Ile Tyr Ser Ser Gln Glu Asp Val Lys Glu Phe Glu Arg
                   405                 410                 415
   GAA GAA ACC CAA GAC AAA GAA GAG AGT GTG GAA TCT AGT TTG CCC CTT   1296
   Glu Glu Thr Gln Asp Lys Glu Glu Ser Val Glu Ser Ser Leu Pro Leu
               420                 425                 430
   AAT GCC ATT GAA CCT TGT GTG ATT TGT CAA GGT CGA CCT AAA AAT GGT   1344
   Asn Ala Ile Glu Pro Cys Val Ile Cys Gln Gly Arg Pro Lys Asn Gly
           435                 440                 445
   TGC ATT GTC CAT GGC AAA ACA GGA CAT CTT ATG GCC TGC TTT ACA TGT   1392
   Cys Ile Val His Gly Lys Thr Gly His Leu Met Ala Cys Phe Thr Cys
       450                 455                 460
   GCA AAG AAG CTA AAG AAA AGG AAT AAG CCC TGC CCA GTA TGT AGA CAA   1440
   Ala Lys Lys Leu Lys Lys Arg Asn Lys Pro Cys Pro Val Cys Arg Gln
   465                 470                 475                 480
   CCA ATT CAA ATG ATT GTG CTA ACT TAT TTC CCC TAG                   1476
   Pro Ile Gln Met Ile Val Leu Thr Tyr Phe Pro  *
                   485                 490
(3) INFORMATIONS POUR LA SEQ ID NO: 2:
   (i) CARACTERISTIQUES DE LA SEQUENCE:
      (A) LONGUEUR: 1182 paires de bases
      (B) TYPE: nucléotide
      (C) NOMBRE DE BRINS: simple
      (D) CONFIGURATION: linéaire
   (ii) TYPE DE MOLECULE: ADNc
   (iii) HYPOTHETIQUE: NON
   (iv) ANTI-SENS: NON
   (ix) CARACTERISTIQUE:
      (A) NOM/CLE: CDS
      (B) EMPLACEMENT:1..1182
   (xi) DESCRIPTION DE LA SEQUENCE: SEQ ID NO: ?:
   ATG GAG GAG CCG CAG TCA GAT CCT AGC GTC GAG CCC CCT CTG AGT CAG      48
   GAA ACA TTT TCA GAC CTA TGG AAA CTA CTT CCT GAA AAC AAC GTT CTG      96
   TCC CCC TTG CCG TCC CAA GCA ATG GAT GAT TTG ATG CTG TCC CCG GAC     144
   GAT ATT GAA CAA TGG TTC ACT GAA GAC CCA GGT CCA GAT GAA GCT CCC     192
   AGA ATG CCA GAG GCT GCT CCC CCC GTG GCC CCT GCA CCA GCA GCT CCT 240
   ACA CCG GCG GCC CCT GCA CCA GCC CCC TCC TGG CCC CTG TCA TCT TCT 288
   GTC CCT TCC CAG AAA ACC TAC CAG GGC AGC TAC GGT TTC CGT CTG GGC 336
   TTC TTG CAT TCT GGG ACA GCC AAG TCT GTG ACT TGC ACG TAC TCC CCT 384
   GCC CTC AAC AAG ATG TTT TGC CAA CTG GCC AAG ACC TGC CCT GTG CAG 432
   CTG TGG GTT GAT TCC ACA CCC CCG CCC GGC ACC CGC GTC CGC GCC ATG 480
   GCC ATC TAC AAG CAG TCA CAG CAC ATG ACG GAG GTT GTG AGG CGC TGC 528
   CCC CAC CAT GAG CGC TGC TCA GAT AGC GAT GGT CTG GCC CCT CCT CAG 576
   CAT CTT ATC CGA GTG GAA GGA AAT TTG CGT GTG GAG TAT TTG GAT GAC 624
   AGA AAC ACT TTT CGA CAT AGT GTG GTG GTG CCC TAT GAG CCG CCT GAG 672
   GTT GGC TCT GAC TGT ACC ACC ATC CAC TAC AAC TAC ATG TGT AAC AGT 720
   TCC TGC ATG GGC GGC ATG AAC CGG AGG CCC ATC CTC ACC ATC ATC ACA 768
   CTG GAA GAC TCC AGT GGT AAT CTA CTG GGA CGG AAC AGC TTT GAG GTG 816
   CGT GTT TGT GCC TGT CCT GGG AGA GAC CGG CGC ACA GAG GAA GAG AAT 864
   CTC CGC AAG AAA GGG GAG CCT CAC CAC GAG CTG CCC CCA GGG AGC ACT    912
   AAG CGA GCA CTG CCC AAC AAC ACC AGC TCC TCT CCC CAG CCA AAG AAG    960
   AAA CCA CTG GAT GGA GAA TAT TTC ACC CTT CAG ATC CGT GGG CGT GAG   1008
   CGC TTC GAG ATG TTC CGA GAG CTG AAT GAG GCC TTG GAA CTC AAG GAT   1056
   GCC CAG GCT GGG AAG GAG CCA GGG GGG AGC AGG GCT CAC TCC AGC CAC   1104
   CTG AAG TCC AAA AAG GGT CAG TCT ACC TCC CGC CAT AAA AAA CTC ATG   1152
   TTC AAG ACA GAA GGG CCT GAC TCA GAC TGA                           1182

## Description

La présente invention concerne une nouvelle méthode de traitement des pathologies hyperprolifératives (cancers, resténoses, etc..) ainsi que les compositions pharmaceutiques correspondantes.

Il est maintenant bien établi qu'une grande majorité des cancers est causée, tout au moins en partie, par des anomalies génétiques qui se traduisent soit par la surexpression d'un ou plusieurs gènes et/ou l'expression d'un ou de gènes mutés ou anormaux. Par exemple, l'expression d'oncogènes génère dans la plupart des cas un cancer. On entend par oncogène un gène génétiquement affecté et dont le produit d'expression perturbe le fonctionnement biologique normal des cellules, initiant ainsi un état néoplasique. Un grand nombre d'oncogènes ont à ce jour été identifiés et partiellement caractérisés comme notamment les gènes *ras, myc, fos, erb, neu, raf, src, fms, jun et abl* dont des formes mutées semblent responsables d'un dérèglement de la prolifération cellulaire

Dans un contexte cellulaire normal, la prolifération de ces oncogènes est vraisemblablement contrée au moins en partie, par la génération de gènes dits supresseurs de tumeurs comme p53 et Rb. Toutefois, certains phénomènes peuvent venir perturber ce mécanisme d'autorégulation cellulaire et favoriser alors le développement d'un état néoplasique. Un de ces évènements consiste en des mutations au niveau des gènes suppresseurs de tumeurs. C'est ainsi que la forme mutée par délétion et/ou mutation du gène p53 est impliquée dans le développement de la plupart des cancers humains (Baker et coll., Science 244 (1989) 217) et que des formes inactivées du gène Rb ont été mises en cause dans différentes tumeurs, et notamment dans les rétinoblastomes ou dans les cancers mésenchymateux comme les ostéosarcomes.

La protéine p53 est une phosphoprotéine nucléaire de 53kD qui est exprimée dans la plupart des tissus normaux. Elle est impliquée dans le contrôle du cycle cellulaire (Mercer et al. Critic Rev. Eucar. Gene Express, 2, 251, 1992), la régulation transcriptionnelle (Fields et al., Sciences (1990) 249, 1046), la réplication de l'ADN (Wilcoq and Lane, (1991), Nature 349, 4290 et Bargonnetti et al., (1992) Cell 65 1083) et l'induction de l'apoptose (Shaw et al., (1992) P.N.A.S.USA 89, 4495). Ainsi, toute exposition de cellules à des agents capables par exemple d'en endommager l'ADN, initie une cascade de signalisation cellulaire qui aboutit à une modification post-transcriptionnelle de la protéine p53 et à l'activation transcriptionnelle par p53 d'un certain nombre de gènes tels gadd45 (growth arrest and DNA damage ) (Kastan et coll. Cell, 71, 587-597,1992 ), p21 WAF/CIP (ElDeiry et coll, Cancer Res., 54, 1169-1174, 1994) ou bien encore mdm2 (mouse double minute) (Barak et coll., EMBO J., 12,461-468,1993).

De ce qui précède, il ressort clairement que l'élucidation des différentes fonctions biologiques de l'ensemble des protéines impliquées notamment dans cette voie de signalisation cellulaire, de leurs modes de fonctionnement et de leurs caractéristiques est d'un intérêt majeur pour la compréhension de la cancérogénèse et la mise au point de méthodes thérapeutiques efficaces dirigées contre le cancer.

La présente invention s'inscrit précisément dans ce contexte en rapportant une nouvelle fonction de la protéine Mdm2.

La protéine Mdm2 est une phosphoprotéine de poids moléculaire de 90kD, exprimée à partir du gène mdm-2 (murine double minute 2). Ce gène mdm2 a été cloné à l'origine dans une cellule tumorale spontanée BALB/c 3T3 et il a été constaté que sa surexpression augmente fortement le pouvoir tumoral (Cahilly-Snyder et coll .Somat.Cell.Mol.Genet.,13,235-244,1987.; Fakharzadeh et coll, EMBO J. 10,1565-1569,1991). Un complexe Mdm2/p53 a été identifié dans plusieurs lignées cellulaires contenant aussi bien une p53 sauvage que des protéines p53 mutées (Martinez et coll., Genes Dev.,5,151-159,1991). En outre, il a été montré que Mdm2 inhibe l'activité transcriptionnelle de p53 sur un promoteur comme celui de la l'activité transcriptionnelle de p53 sur un promoteur comme celui de la créatine Kinase de muscle indiquant que Mdm2 peut réguler l'activité de p53 (Momand et coll., Cell, 69,1237-1245,1992 ; Oliner et coll. Nature, 362,857-860, 1993). Le document W093/20238 décrit un test de diagnostic basé sur la surexpression du gène mdm2. Ce document décrit aussi le principe de l'utilisation de composés capables d'interférer dans la liaison Mdm-2 / p53 afin de prévenir la séquestration de p53 par Mdm-2.

Au vu de l'ensemble de ces résultats, la protéine Mdm2 est donc à ce jour essentiellement reconnue comme un modulateur des activités de p53. En complexant les protéines p53 sauvages ou mutées, elle inhibe leur activité transcriptionnelle et contribue de cette manière à la dérégulation de la prolifération cellulaire. Par conséquent, l'exploitation sur un plan thérapeutique de ces informations consiste majoritairement à rechercher des moyens pour s'opposer à ce blocage par Mdm2 de la protéine p53.

De manière inattendue, la demanderesse a mis en évidence que cette protéine Mdm2 possèdait un caractère oncogénique propre, c'est-à-dire totalement distinct de celui associé à sa forme complexée avec la protéine p53. Plus précisément, la protéine Mdm2 développe des propriétés ôncogéniques dans un contexte p53 nul. Pour appuyer cette découverte à savoir que les propriétés oncogéniques de Mdm-2 sont indépendantes de p53 et en particulier ne résultent pas de l'inhibition de l'activité transactivatrice de p53 sauvage, nous avons montré qu'un mutant de p53 (p53 (14-19) ; Lin et al., Genes Dev., 1994, 8, 1235-1246) qui a conservé ses propriétés transactivatrices mais qui n'interagit plus avec Mdm-2 est incapable de bloquer les propriétés oncogéniques de Mdm-2. Il est également montré que Mdm-2 et en particulier le domaine 1-134 de Mdm-2 est capable de débloquer un arrêt du cycle cellulaire en G1 induit par la surexpression de p107. Mdm-2 s'avère donc être un régulateur important de facteurs impliqués dans le contrôle du cycle cellulaire, autres que p53.

La présente invention résulte en partie de la mise en évidence que la séquence protéique 1-134 de la séquence identifiée en SEQ ID N°1, de la protéine Mdm2 est suffisante pour traduire le potentiel oncogénique de ladite protéine.

Elle résulte également de la mise en évidence qu'il est possible d'affecter ce caractère oncogénique de la protéine Mdm2 en utilisant des composés capables d'interagir avec elle. La présente invention décrit également des systèmes particulièrement efficaces permettant la délivrance in vivo, directement dans les tumeurs, de tels composés et ainsi de lutter contre le développement des cancers. La présente invention offre ainsi une nouvelle approche particulièrement efficace pour le traitement des tumeurs en particulier à contexte p53 nul telles que les cancers suivants les adénocarcinomes du colon, les cancers de la thyroïde, les carcinomes du poumon, les leucémies myéloïdes, les cancers colorectaux, les cancers du sein, les cancers du poumon, les cancers gastriques, les cancers de l'oesophage, les lymphômes B, les cancers ovariens, les cancers de la vessie, les glioblastomes, etc...

Un premier objet de l'invention réside donc dans l'utilisation d'un composé capable d'antagoniser au moins partiellement l'activité oncogénique de la protéine Mdm2 pour la préparation d'une composition pharmaceutique destinée au traitement des cancers à contexte p53 nul.

Au sens de l'invention, on entend par cancer à contexte p53 nul, un cancer où p53 serait incapable d'exercer ses fonctions de gène suppresseur de tumeur par toute modification ou tout mécanisme autre que la fixation de Mdm-2 sur p53, cette fixation empêchant p53 de jouer son rôle de suppresseur de tumeur et permettant aux cellules d'échapper à une croissance régulée par p53. On peut citer de façon non exhaustive parmi ces modifications ou mécanismes bloquant l'activité de suppresseur de tumeur de p53, par exemple, des altérations génétiques du gène p53 (mutations ponctuelles, délétions, etc), l'interaction avec d'autres protéines que Mdm-2, la dégradation protéolytique très rapide de la protéine p53 liée à la présence de la protéine E6 des virus des papillomes humains à haut risque tels que HPV-16 et HPV-18, etc.

Au sens de l'invention, l'inhibition de l'activité oncogénique de la protéine Mdm2 peut être obtenue selon deux méthodes.

Elle est préférentiellement accomplie en intervenant directement au niveau du domaine 1-134 de celle-ci. C'est ainsi que toute protéine capable de se lier à ce domaine aura un rôle antagoniste sur les propriétés oncogéniques de Mdm2.

Toutefois, cet effet inhibiteur peut également être atteint via l'interaction d'un composé avec un domaine voisin, comme par exemple le domaine 135-491 de mdm2, représenté sur la séquence SEQ ID N°1 ou sa séquence C terminale représentée sur la séquence SEQ ID N°1. En conséquence, la présente invention vise en outre l'utilisation de tout composé qui, bien que n'interagissant pas directement avec ce domaine, est néammoins capable d'en affecter le caractère oncogénique.

Selon un mode particulier, la présente invention vise l'utilisation d'un composé capable de se lier au niveau du domaine 1-134 de la séquence représentée en SEQ ID N° 1 de la protéine Mdm2 en vue de préparer une composition pharmaceutique destinée au traitement des cancers à contexte p53 nul.

A titre de composé susceptible d'interagir directement au niveau du domaine 1-134, de la protéine Mdm2, on peut citer plus particulièrement les scFV dirigés spécifiquement contre ce domaine.

Les ScFv sont des molécules ayant des propriété de liaison comparables à celles d'un anticorps et actives intracellulairement. Il s'agit plus particulièrement de molécules constituées d'un peptide correspondant au site de liaison de la région variable de la chaine légère d'un anticorps relié par un linker peptidique à un peptide correspondant au site de liaison de la région variable de la chaine lourde d'un anticorps. Il a été montré par la demanderesse que de tels ScFv pouvaient être produits in vivo par transfert de gène (Cf demande WO 94/29446).

Il peut également s'agir de peptides ou de protéines déjà connus pour leur aptitude à se lier spécifiquement avec le domaine 1-134 de Mdm2 comme par exemple tout ou partie du domaine de liaison de la protéine p53 avec la SEQ ID N°1 et plus particulièrement tout ou partie d'un des peptides 1-52, 1-41 et 6-41 de la séquence de p53 représentée en SEQ ID N°2, (Oliner et al., Nature, 1993, 362, 857-860) ou plus simplement tout ou partie du peptide 16-25 cartographié plus précisément (Lane et al., Phil. Trans. R. Soc. London B., 1995, 347, 83-87), ou même les peptides 18-23 des p53 humaine ou murine, ou encore des peptides dérivés proches de ceux précédemment cités dans lesquels les résidus critiques pour l'interaction avec Mdm-2 auront été conservés (Picksley et al., Oncogene, 1994, 9, 2523-2529).

Il peut également être mis en oeuvre selon l'invention des composés de se lier à des domaines voisins du domaine 1-134 de Mdm2 représenté en SEQ ID N°1 et affectant de part cette liaison l'activité oncogénique de la protéine Mdm2. A ce titre, on peut citer ceux interagissant au niveau du domaine C terminal de ladite protéine comme par exemple les facteurs transcriptionnels TFII, TBP, et TaF250 de même que les protéines interagissant au niveau du domaine 135-491 de Mdm2 représenté en SEQ ID N°1 comme par exemple les protéines L5 (protéine ribosomale) et Rb (protéine rétinoblastome) et le facteur transcriptionnel E2F ( régulé par Rb).

Un autre objet de la présente invention vise également l'utilisation de scFV dirigés spécifiquement contre ce domaine 1-134 de la séquence représentée en SEQ ID N° 1 de la protéine Mdm2 en vue de préparer une composition pharmaceutique destinée au traitement des cancers.

Au sens de l'invention, il est entendu que l'ensemble des interactions citées ci-dessus affectent de manière conséquente le caractère oncogénique de Mdm2. En outre, ces protéines peuvent être utilisées, en tout ou partie, dans la mesure où il est mis en oeuvre leur partie active vis-à-vis d'un des domaines de liaison avec la protéine Mdm2 et que cette interaction conduit à une affectation du caractère oncogénique de celle-ci.

Dans le cadre de la présente invention, ces composés peuvent être utilisés tels quels ou, avantageusement, sous forme de constructions génétiques permettant leur expression in vivo.

Un mode de réalisation particulièrement avantageux de la présente invention consiste à mettre en oeuvre une séquence nucléique codant pour un composé capable d'antagoniser au moins partiellement l'activité oncogénique de la protéine Mdm2 pour la préparation d'une composition pharmaceutique destinée au traitement des cancers à contexte p53 nul.

Dans cette perspective, les acides nucléiques utilisés dans le cadre de l'invention peuvent être de différents types. Il s'agit de manière préférentielle :
- d'acides nucléiques antisens,
- d'oligoribonucléotides capables de fixer directement l'un des domaines de la protéine Mdm2 et d'inhiber son activité oncogénique (oligonucléotide ligand),
- d'acides nucléiques codant en tout ou partie pour des peptides ou protéines capables de s'oligomériser avec l'un des domaines de Mdm2 et d'inhiber son activité oncogénique,
- d'acides nucléiques codant pour des anticorps intracellulaires (par exemple des fragments variables à chaîne unique issu d'un anticorps) dirigés contre le domaine 1-134 de la séquence SEQ ID N°1 de la protéine Mdm2.

Selon un mode particulier de la présente invention, l'acide nucléique est un acide nucléique antisens. Cet antisens est un ADN codant pour un ARN complémentaire de l'acide nucléique codant pour la protéine Mdm2 et capable de bloquer sa transcription et/ou sa traduction (ARN antisens) ou un ribozyme.

Plus récemment, un nouveau type d'acides nucléiques capables de réguler l'expression de gènes-cible a été mis en évidence. Ces acides nucléiques ne s'hybrident pas avec les ARNm cellulaires, mais directement avec l'ADN génomique en double-brin. Cette nouvelle approche repose sur la mise en évidence que certains acides nucléiques sont capables d'interagir spécifiquement dans le grand sillon de la double-hélice d'ADN pour former localement des triple-hélices, conduisant à une inhibition de la transcription de gènes-cible. Ces acides nucléiques reconnaissent sélectivement la double-hélice d'ADN au niveau de séquences oligopurine.oligopyrimidine, c'est-à-dire au niveau de régions possédant une séquence oligopurique sur un brin et une séquence oligopyrimidique sur le brin complémentaire, et y forment localement une triple-hélice. Les bases du troisième brin (l'oligonucléotide) forment des liaisons hydrogène (liaisons Hoogsteen ou Hoogsteen inverse) avec les purines des paires de bases Watson-Crick. De tels acides nucléiques ont notamment été décrits par le Pr. Hélène dans Anti-Cancer drug design 6 (1991) 569.

Les acides nucléiques antisens selon la présente invention peuvent être des séquences d'ADN codant pour des ARN antisens ou pour des ribozymes. Les ARN antisens ainsi produits peuvent interagir avec un ARNm ou un ADN génomique cible et former avec celui-ci des doubles ou des triples hélices. Il peut également s'agir de séquences (oligonucléotides) antisens, éventuellement modifiés chimiquement, capables d'interagir directement avec le gène ou l'ARN cible.

Toujours selon un mode préféré de mise en oeuvre de la présente invention, l'acide nucléique est un oligonucléotide antisens tel que défini précédemment, éventuellement modifié chimiquement. Il peut s'agir en particulier d'oligonucléotides dont le squelette phosphodiester a été modifié chimiquement, tels que par exemple les oligonucléotides phosphonates, phosphotriesters, phosphoramidates et phosphorothioates qui sont décrits, par exemple, dans la demande de brevet WO94/08003. Il peut également s'agir d'oligonucléotides alpha, ou d'oligonucléotides conjugués à des agents tels que des composés acrylants.

Au sens de la présente invention, on entend par oligonucléotide ligand, un oligoribonucléotide ou un oligodéoxyribonucléotide capable de se fixer spécifiquement à la protéine Mdm-2 afin d'inhiber sa fonction oncogénique. De tels nucléotides peuvent par exemple être mis en évidence par des techniques d'"évolution in vitro" comme par exemple la technique SELEX (Edgington, Bio/technology, 1992, 10, 137-140 ; Brevets US 5,270,163 et WO 91/19813).

Plus généralement, ces acides nucléiques peuvent être d'origine humaine, animale, végétale, bactérienne, virale, synthétique, etc. Us peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques.

Comme indiqué plus loin, ils peuvent par ailleurs être incorporés dans des vecteurs, tels que des vecteurs plasmidiques, viraux ou chimiques. Ils peuvent également être administrés tels quels, sous forme d'ADN nu selon la technique décrite dans la demande WO 90/11092 ou sous forme complexée, par exemple avec du DEAE-dextran (Pagano et al., J.Virol. 1 (1967) 891), avec des protéines nucléaires (Kaneda et al., Science 243 (1989) 375), avec des lipides ou polymères cationiques (Felgner et al., PNAS 84 (1987) 7413), sous forme de liposomes (Fraley et al., J.Biol.Chem. 255 (1980) 10431), etc.

Préférentiellement, la séquence utilisée dans le cadre de l'invention fait partie d'un vecteur. L'emploi d'un tel vecteur permet en effet d'améliorer l'administration de l'acide nucléique dans les cellules à traiter, et également d'augmenter sa stabilité dans lesdites cellules, ce qui permet d'obtenir un effet thérapeutique durable. De plus, il est possible d'introduire plusieurs séquences d'acide nucléique dans un même vecteur, ce qui augmente également l'efficacité du traitement.

Le vecteur utilisé peut être d'origine diverses, dès lors qu'il est capable de transformer les cellules animales, de préférence les cellules cancéreuses humaines. Dans un mode préféré de mise en oeuvre de l'invention, on utilise un vecteur viral, qui peut être choisi parmi les adénovirus, les rétrovirus, les virus adéno-associés (AAV) ou le virus de l'herpès.

A cet égard, la présente invention a également pour objet tout vecteur viral comprenant, inséré dans son génome, un acide nucléique codant pour un composé capable d'antagoniser au moins partiellement le caractère oncogénique de la protéine Mdm2.

Plus particulièrement, elle concerne tout virus recombinant comprenant une séquence d'acides nucléiques codant pour un composé capable de se lier à la protéine Mdm2 de manière à affecter son potentiel oncogénique. Dans ce contexte, la séquence d'acides nucléiques peut coder pour l'un des peptides, protéines ou facteurs transcriptionnels précédemment identifiés.

Plus préférentiellement, cette séquence d'acides nucléiques code pour un scFv ou un peptide capable d'interagir au niveau du domaine 1-134 (SEQ ID N°1) de la protéine Mdm2.

Avantageusement, les virus utilisés dans le cadre de l'invention sont préférentiellement défectifs, c'est-à-dire qu'ils sont incapables de se répliquer de façon autonome dans la cellule infectée. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence codant pour le composé possédant un rôle antagoniste sur les propriétés oncogéniques de la protéine Mdm2. Préférentiellement, le virus défectif conserve néanmoins les séquences de son génome qui sont nécessaires à l'encapsidation des particules virales.

S'agissant plus particulièrement d'adénovirus, différents sérotypes, dont la structure et les propriétés varient quelque peu, ont été caractérisés. Parmi ces sérotypes, on préfère utiliser dans le cadre de la présente invention les adénovirus humains de type 2 ou 5 (Ad 2 ou Ad 5) ou les adénovirus d'origine animale (voir demande FR 93 05954). Parmi les adénovirus d'origine animale utilisables dans le cadre de la présente invention on peut citer les adénovirus d'origine canine, bovine, murine, (exemple : Mav1, Beard et al., Virology 75 (1990) 81), ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Préférentiellement, les adénovirus défectifs de l'invention comprenent les ITR, une séquence permettant l'encapsidation et la séquence codant pour le modulateur des calpaïnes. Encore plus préférentiellement, dans le génome des adénovirus de l'invention, le gène E1 et au moins l'un des gènes E2, E4, L1-L5 sont non fonctionnels. Le gène viral considéré peut être rendu non fonctionnel par toute technique connue de l'homme du métier, et notamment par suppression totale, substitution, délétion partielle, ou addition d'une ou plusieurs bases dans le ou les gènes considérés. De telles modifications peuvent être obtenues in vitro (sur de l'ADN isolé) ou in situ, par exemple, au moyens des techniques du génie génétique, ou encore par traitement au moyen d'agents mutagènes.

Les adénovirus recombinants défectifs selon l'invention peuvent être préparés par toute technique connue de l'homme du métier (Levrero et al., Gene 101 (1991) 195, EP 185 573 ; Graham, EMBO J. 3 (1984) 2917). En particulier, ils peuvent être préparés par recombinaison homologue entre un adénovirus et un plasmide portant entre autre la séquence d'ADN codant pour l'inhibiteur des ETS. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). Des stratégies de construction de vecteurs dérivés des adénovirus ont également été décrites dans les demandes n° FR 93 05954 et FR 93 08596.

Ensuite, les adénovirus qui se sont multipliés sont récupérés et purifiés selon les techniques classiques de biologie moléculaire, comme illustré dans les exemples.

Concernant les virus adéno-associés (AAV), il s'agit de virus à ADN de taille relativement réduite, qui s'intègrent dans le génome des cellules qu'ils infectent, de manière stable et site-spécifique. Ils sont capables d'infecter un large spectre de cellules, sans induire d'effet sur la croissance, la morphologie ou la différenciation cellulaires. Par ailleurs, ils ne semblent pas impliqués dans des pathologies chez l'homme. Le génome des AAV a été cloné, séquencé et caractérisé. Il comprend environ 4700 bases, et contient à chaque extrémité une région répétée inversée (ITR) de 145 bases environ, servant d'origine de réplication pour le virus. Le reste du génome est divisé en 2 régions essentielles portant les fonctions d'encapsidation : la partie gauche du génome, qui contient le gène rep impliqué dans la réplication virale et l'expression des gènes viraux; la partie droite du génome, qui contient le gène cap codant pour les protéines de capside du virus.

L'utilisation de vecteurs dérivés des AAV pour le transfert de gènes in vitro et in vivo a été décrite dans la littérature (voir notamment WO 91/18088; WO 93/09239; US 4,797,368, US5,139,941, EP 488 528). Ces demandes décrivent différentes constructions dérivées des AAV, dans lesquelles les gènes rep et/ou cap sont délétés et remplacés par un gène d'intérêt, et leur utilisation pour transférer in vitro (sur cellules en culture) ou in vivo (directement dans un organisme) ledit gène d'intérêt. Les AAV recombinants défectifs selon l'invention peuvent être préparés par co-transfection, dans un lignée cellulaire infectée par un virus auxiliaire humain (par exemple un adénovirus), d'un plasmide contenant la séquence codant pour l'inhibiteur des ETS bordé de deux régions répétées inversées (ITR) d'AAV, et d'un plasmide portant les gènes d'encapsidation (gènes rep et cap) d'AAV. Les AAV recombinants produits sont ensuite purifiés par des techniques classiques.

Concernant les virus de l'herpès et les rétrovirus, la construction de vecteurs recombinants a été largement décrite dans la littérature : voir notamment Breakfield et al., New Biologist 3 (1991) 203 ; EP 453242, EP 178220, Bernstein et al. Genet. Eng. 7 (1985) 235 ; McCormick, BioTechnology 3 (1985) 689, etc. En particulier, les rétrovirus sont des virus intégratifs, infectant sélectivement les cellules en division. Ils constituent donc des vecteurs d'intérêt pour des applications cancer. Le génome des rétrovirus comprend essentiellement deux LTR, une séquence d'encapsidation et trois régions codantes (gag, pol et env). Dans les vecteurs recombinants dérivés des rétrovirus, les gènes gag, pol et env sont généralement délétés, en tout ou en partie, et remplacés par une séquence d'acide nucléique hétérologue d'intérêt. Ces vecteurs peuvent être réalisés à partir de différents types de rétrovirus tels que notamment le MoMuLV ("murine moloney leukemia virus" ; encore désigné MoMLV), le MSV ("murine moloney sarcoma virus"), le HaSV ("harvey sarcoma virus") ; le SNV ("spleen necrosis virus") ; le RSV ("rous sarcoma virus") ou encore le virus de Friend.

Pour construire des rétrovirus recombinants comportant une séquence d'intérêt, un plasmide comportant notamment les LTR, la séquence d'encapsidation et ladite séquence d'intérêt est généralement construit, puis utilisé pour transfecter une lignée cellulaire dite d'encapsidation, capable d'apporter en trans les fonctions rétrovirales déficientes dans le plasmide. Généralement, les lignées d'encapsidation sont donc capables d'exprimer les gènes gag, pol et env. De telles lignées d'encapsidation ont été décrites dans l'art antérieur, et notamment la lignée PA317 (US 4,861,719) ; la lignée PsiCRIP (WO 90/02806) et la lignée GP+envAm-12 (WO 89/07150). Par ailleurs, les rétrovirus recombinants peuvent comporter des modifications au niveau des LTR pour supprimer l'activité transcriptionnelle, ainsi que des séquences d'encapsidation étendues, comportant une partie du gène gag (Bender et al., J. Virol. 61 (1987) 1639). Les rétrovirus recombinants produits sont ensuite purifiés par des techniques classiques.

Avantageusement, dans les vecteurs de l'invention, la séquence codant pour le composé possédant des propriétés antagonistes sur le caractère oncogénique de Mdm2 est placée sous le contrôle de signaux permettant son expression dans les cellules tumorales. Préférentiellement, il s'agit de signaux d'expression hétérologues, c'est-à-dire de signaux différents de ceux naturellement responsables de l'expression de l'inhibiteur. Il peut s'agir en particulier de séquences responsables de l'expression d'autres protéines, ou de séquences synthétiques. Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris du virus utilisé. A cet égard, on peut citer par exemple les promoteurs E1A, MLP, CMV, LTR-RSV, etc. En outre, ces séquences d'expression peuvent être modifiées par addition de séquences d'activation, de régulation, ou permettant une expression tissu-spécifique. Il peut en effet être particulièrement intéressant d'utiliser des signaux d'expression actifs spécifiquement ou majoritairement dans les cellules tumorales, de manière à ce que la séquence d'ADN ne soit exprimée et ne produise son effet que lorsque le virus a effectivement infecté une cellule tumorale.

Dans un mode particulier de réalisation, l'invention concerne un virus recombinant défectif comprenant une séquence d'ADNc codant pour un composé possédant des propriétés antagonistes sur le caractère oncogénique de Mdm2 sous le contrôle d'un promoteur viral, choisi de préférence parmi le LTR-RSV et le promoteur CMV.

Toujours dans un mode préféré, l'invention concerne un virus recombinant défectif comprenant une séquence d'ADN codant pour un composé possédant des propriétés antagonistes sur le caractère oncogénique de Mdm2 sous le contrôle d'un promoteur permettant une expression majoritaire dans les cellules tumorales.

L'expression est considérée comme majoritaire au sens de l'invention lorsque, même si une expression résiduelle est observée dans d'autres types cellulaires, les niveaux d'expression sont supérieurs dans les cellules tumorales.

La présente invention s'étend également à l'utilisation d'une séquence nucléique codant pour des anticorps intracellulaires ou encore scFV, dirigés contre le domaine 1-134 de la séquence de la protéine Mdm2 représentée en SEQ ID N°1 pour la préparation d'une composition pharmaceutique destinée de manière générale au traitement du cancer.

Elle concerne également toute composition pharmaceutique comprenant un composé capable d'inhiber l'activité oncogénique de la protéine Mdm2, ou une séquence d'acides nucléiques codant pour un tel composé. Selon un mode particulier de réalisation de l'invention cette composition comprend un ou plusieurs virus recombinants défectifs tels que décrits précédemment. Ces compositions pharmaceutiques peuvent être formulées en vue d'administrations par voie topique, orale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe dans la tumeur du patient. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. L'injection directe dans la tumeur du patient est avantageuse car elle permet de concentrer l'effet thérapeutique au niveau des tissus affectés.

Les doses de virus recombinant défectif utilisées pour l'injection peuvent être adaptées en fonction de différents paramètres, et notamment en fonction du vecteur viral, du mode d'administration utilisé, de la pathologie concernée ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature. Concernant les rétrovirus, les compositions selon l'invention peuvent comporter directement les cellules productrices, en vue de leur implantation.

Les compositions pharmaceutiques selon l'invention sont particulièrement avantageuses pour neutraliser l'activité oncogénique des protéines Mdm2 et de ce fait pour moduler la prolifération de certains types cellulaires.

En particulier, ces compositions pharmaceutiques sont appropriées au traitement de cancers possédant un p53 nul comme par exemple les cancers suivants: les adénocarcinomes du colon, les cancers de la thyroïde, les carcinomes du poumon, les leucémies myéloïdes, les cancers colorectaux, les cancers du sein, les cancers du poumon, les cancers gastriques, les cancers de l'oesophage, les lymphômes B, les cancers ovariens, les cancers de la vessie, les glioblastomes, etc.

La présente invention est avantageusement utilisée in vivo pour la destruction de cellules en hyperprolifération (i.e. en prolifération anormale). Elle est ainsi applicable à la destruction des cellules tumorales ou des cellules de muscle lisse de la paroi vasculaire (resténose).

D'autres avantages de la présente invention apparaîtront à la lecture des exemples et figures qui suivent, qui doivent être considérés comme illustratifs et non limitatifs.
- Figure 1 :: Représentation des protéines Mdm-2 de A à F.
- Figure 2 :: Graphe de la transfection de cellules Saos-2 avec des plasmides exprimant diverses protéines Mdm-2
- Figure 3 :: Représentation schématique de l'inhibition des propriétés transformantes de Mdm2 par différents p53.
- Figure 4 :: Effet d'une surexpression Mdm2 sur le cycle cellulaire.
- Figure 5 :: Effet d'une surexpression Mdm2 sur le cycle cellulaire.

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondament décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Pour les ligatures, les fragments d' ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant. L'amplification d'ADN génomique est réalisée plus particulièrement dans les conditions suivantes : 5 minutes à 100°C, 30 cycles d'une minute à 95°C, 2 minutes à 58°C puis 3 minutes à 72°C au moyen de sondes appropriées. Les produits d'amplification sont analysés par électrophorèse sur gel.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Matériels et Méthodes :

### 1.Construction utilisées:

- le plasmide pBKCMV est commercialisée par Stratagène et contient le gène de résistance à la néomycine.
- les plasmides pC53C1N3 et p53-4.2. N3 codant respectivement pour p53 sauvage et pour p53 R273H proviennent de A. Levine (Hinds et. al.,. Cell Growth and Diff. (1990), 1, 571).
- le plasmide pBKp53 (R273H) contient le minigène humain p53. Il a été obtenu à partir de pC53-4.2. N3.
- le plasmide pBKMdm2 a été obtenu par clonage dans pBKCMV d'une cassette codante consistant en la région non traduite de l'extrémité de la séquence codant pour la β globine suivie de la séquence codant pour mdm2.
- le plasmide pGKhygro exprime le gène de résistance à l'hygromycine (Nature (1990) 348, 649-651).
- le plasmide pCMVNeoBam permettant l'expression du gène de résistance à la néomycine (Hinds et.al. (1990) Cell. Growth and Diff., 1,571-580).
- .les plasmides pCMVp107 et pCMVCD20 permettant l'expression de la protéine p107 et du marqueur de surface CD20 (Zhu et.al. (1993) Genes and Development, 7,1111-1125).
- les plasmides pCMVE2F-4 et pCMVE2F-5 permettant l'expression des protéines E2F-4 et E2F-5 (Sardet et.al. (1995) Proc. Natl. Acad. Sc., 92, 2403-2407).
- les plasmides pLexA, pLexA(6-41), pLexA(16-25) permettant l'expression du domaine de fixation à l'ADN de LexA (aa 1 à 87) libre ou fusionné en phase avec p53(6-41) ou p53 (16-25). pLexA(6-41) et pLexA(16-25) ont été obtenus à partir du plasmide pLexApolyII construit au LGME (Strasbourg).
- les plasmides d'expression eucaryote de p107 : p107(385-1068), p107(1-781) et p107(781-1068) (Zhu et al., EMBO J. 14 (1995) 1904),
- le plasmide pSGK1HAp107 permet l'expression in vitro et in vivo de p107. p107 est dans le contexte d'une séquence Kozak et l'épitope HA est exprimé en fusion à l'extrémité C-terminale de p107.
- les plasmides pBC-MDM2 et pBC-MDM2(1-134) ont été obtenus par clonage de MDM2 et MDM2(1-134) dans pBC (Chatton et al., Biotechniques 18 (1995) 142).
- les plasmides pGex-MDM2 et pGex-MDM2(1-177) ont été obtenus par clonage de MDM2 et MDM2(1-177) dans pGex.

### 2.Méthode:

L'expression de p53 est déterminée par Western Blotting sur l'extrait cellulaire entier à l'aide d'un anticorps monoclonal D01.

L'expression de l'ARMm codant pour la protéine Mdm2 est estimée par RT-PCR semi-quantitative.

L'absence de contamination de l'ADN est vérifiée par PCR.

### Exemple 1 : Mise en évidence des propriétés transformantes de mdm2.

Des cellules Saos-2 sont transfectées avec soit un plasmide pBKMDM2, un plasmide témoin pBKp53 (R273H) ou un plasmide témoin en p53 négatif pBKCMV, puis sélectionnées pour la résistance à la Geneticine 418(G418).

Dans un premier essai, des clones sont sélectionnés individuellement et propagé alors que dans les 2 autres essais, les clones non isolés sont mis en culture dans un milieu agar soft.

Pour cela, 10⁴ cellules sont ensemencées en duplicata dans 0,375 % de soft agar. Après 24 heures, on détermine le nombre total de colonies avec plus de 50 celllules ainsi que le nombre de cellules par colonie (taille des colonies). Chaque valeur donnée correspond à une moyenne de quatre expériences réalisées en double. Les résultats obtenus sont présentés en tableau I. Les clones dans l'essai n°1 correspondants à mdm2 sont identifiés sous M1 à M6, ceux de p53 (R273H) sous p53-1 à p53-6 et ceux du témoin sous Co1 à Co5).

Comme attendu, Co1 et Co4 n'expriment pas le mdm2 transfecté et Co 1-3 la protéine p53.

**TABLEAU I**

| | | | **CROISSANCE SUR MILIEU SOFT AGAR COLONIES** **(TAILLE)** | **EXPRESSION** | |
|---|---|---|---|---|---|
| | | | | **MDM2** | **P53** |
| **EXPERIENCE 1** (clones isolés) | **MDM2** | M1 | 634 (100-600) | + | ND |
| | | M2 | 594(100-600) | ++ | ND |
| | | M3 | 460 (100-600) | + | ND |
| | | M4 | 310 (50-500) | ++ | ND |
| | | M5 | 57 (50-200) | +/- | ND |
| | | M6 | 23 (50) | + | ND |
| | **Contrôle** | Co1 | 97 (50-200) | - | - |
| | | Co2 | 68 (50-200) | ND | - |
| | | Co3 | 35 (50-100) | ND | - |
| | | Co4 | 11(50) | - | ND |
| | | Co5 | 4 (50) | ND | ND |
| | **p53R** **(273)H** | p53-1 | 190 (50-600) | ND | +++ |
| | | p53-2 | 137 (50-300) | ND | ++++ |
| | | p53-3 | 88 (50-200) | ND | +++ |
| | | p53-4 | 53 (50-200) | ND | +++ |
| | | p53-5 | 47 (50-200) | ND | ++ |
| | | p53-6 | 38 (50-100) | ND | + |
| **EXPERIENCE 2** | **MDM2** | M-P1 | 395 (100-1000) | ++ | ND |
| | **Contrôle** | Co-P1 | 21 (50-200) | ND | ND |
| | | Co-P2 | 18 (50-200) | ND | ND |
| **EXPERIENCE 3** | **MDM2** | M-P1 | 255 (50-300) | ND | ND |
| | | M-P2 | 220 (50-300) | ND | ND |
| | | CoP1 | 110 (50-200) | ND | ND |
| | **Contrôle** | Co-P2 | 110 (50-200) | ND | ND |
| | | Co-P3 | 100 (50-200) | ND | ND |
| | | Co-P4 | 75 (50-200) | ND | ND |

### Exemple 2: La région N-terminale de Mdm-2 (1-134) séq ID N°1 est nécessaire et suffisante pour stimuler la croissance en agar soft des cellules Saos-2.

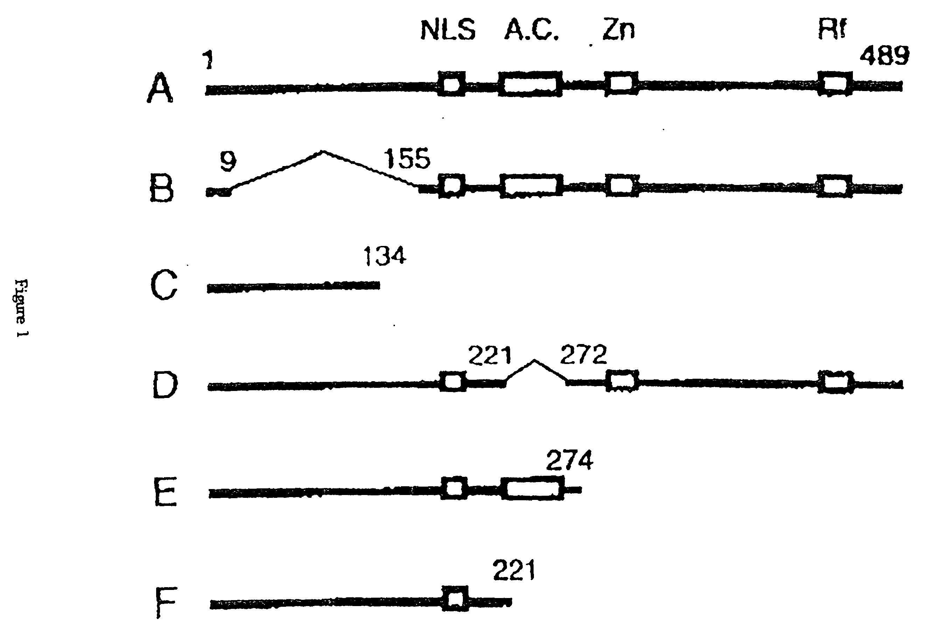

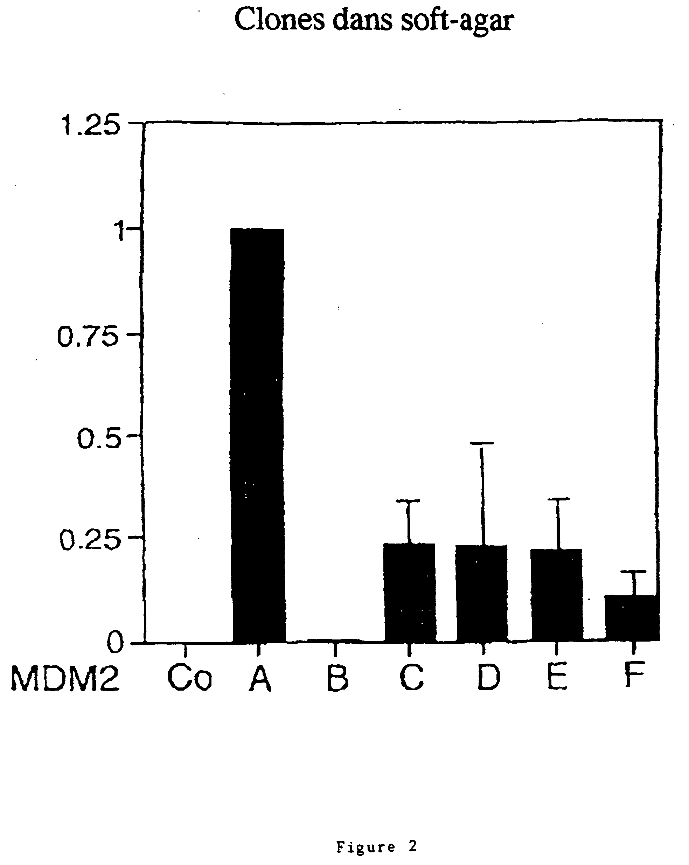

Des cellules Saos-2 sont transfectées avec soit des plasmides pBKCMV qui expriment à la fois la résistance neo et les protéines mdm-2 de A à F décrites dans la figure 1, soit un plasmide témoin pBKCMV vide, puis sélectionnées pour la résistance à la G418. Les cellules survivantes sont regroupées, amplifiées puis testées pour la formation de colonies en agar soft. Les résultats de la figure 2 sont exprimés en nombre de clones formés en agar soft relatif à celui avec mdm-2 entier (A). Ces résultats sont issus de deux expériences représentatives de transfection indépendantes dans lesquelles entre 3 et 7 pools de cellules différents ont été testés, suivant la construction. Ils montrent clairement que le domaine N-terminal de mdm-2 possède des propriétés oncogéniques. La construction la plus efficace correspond à la protéine entière.

### Exemple 3: Réversion des propriétés oncogéniques de Mdm-2 par la p53 sauvage, des mutants de p53 et des fragments de p53.

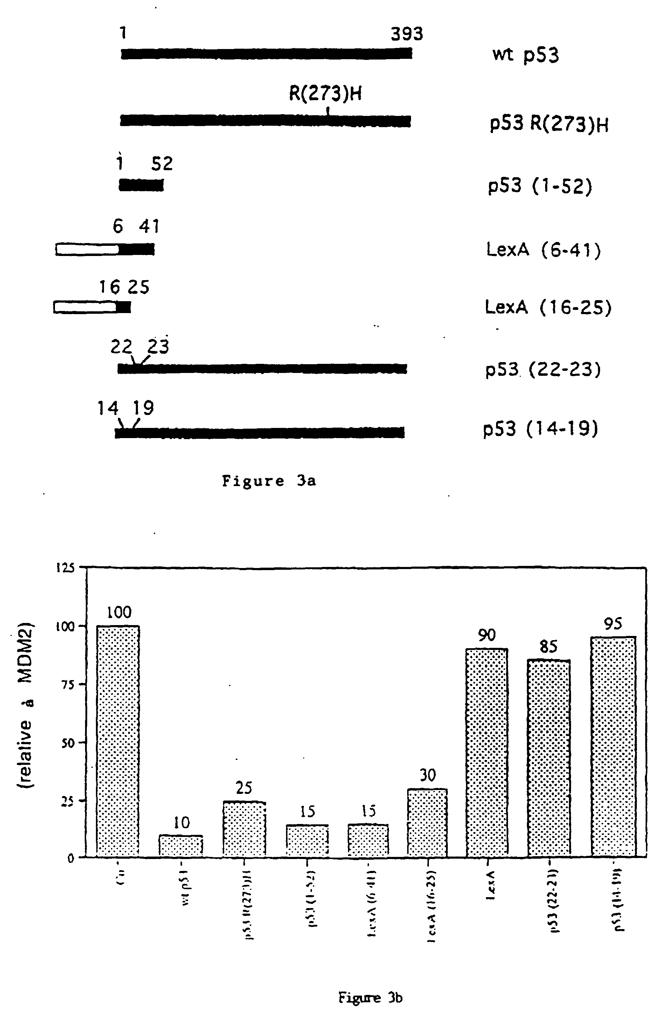

Un lot de cellules Saos-2 transformées par Mdm-2 est co-transfecté avec le plasmide pGKhygro et soit pC53C1N3 (p53) pC53-4.2N3 p53(R(273)H, p53 (1-52), pLexA(6-41), pLexA(16-25), pLexA, p53(L14Q,F19S), p53(L22Q,W23S), soit pCMVNeoBam, puis sélectionnées pour la résistance à l'hygromycine en présence de G418. 10 0000 cellules provenant de 3 à 5 pools indépendants de cellules résistantes sont ensemencées en duplicate en agar soft (0,375 %). Après 25 jours de culture, les colonies contenant au moins 50 cellules sont comptées. La figure 3 présente les résultats d'une expérience représentative et donne une représentation schématique des différentes p53 testées pour inhiber les propriétés transformantes de Mdm-2. Il ressort de cette expérience que seules les constructions permettant l'expression de protéines capables de se lier à la protéine mdm-2, en l'occurence p53, p53 R273H, p53(1-52), LexA(6-41), LexA(16-25) inhibent les propriétés oncogéniques de Mdm-2. En revanche, les double mutants dont il est démontré qu'ils ont perdu la capacité de liaison à mdm-2 (Lin et al., Gene Dev., 1994, 8, 1235-1246) n'ont pas d'effet inhibiteur. Le fait que le mutant p53(14-19) qui a conservé les propriétés transactivatrices de la p53 sauvage n'inhibe pas la transformation par Mdm-2 confirment que les propriétés oncogéniques de Mdm-2 sont indépendantes de l'inhibition par Mdm-2 des propriétés transactivatrices de p53.

### Exemple 4: Mdm-2 inhibe le blocage en G1 du cycle cellulaire induit par p107 dans les cellules Saos-2.

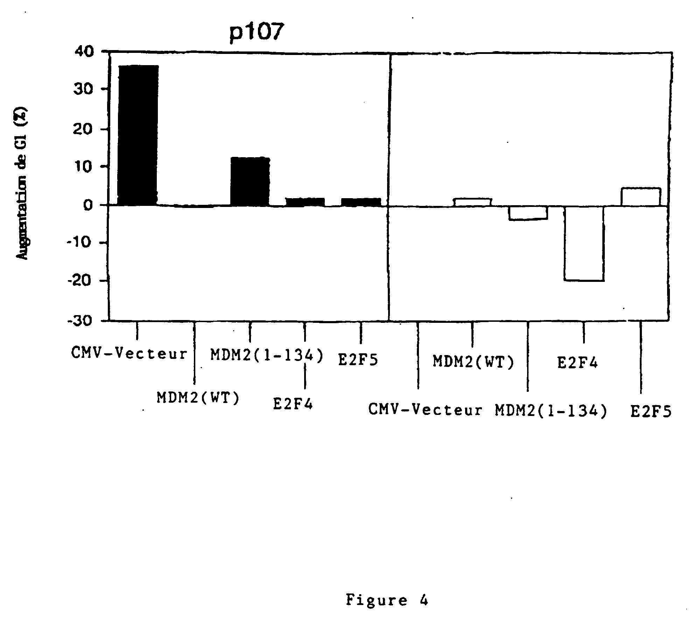

Des cellules Saos-2 sont co-transfectées avec trois types de plasmides, (i) un plasmide pour l'expression de CD-20 (pCMVCD20, 2 µg, codant pour le marqueur de surface cellulaire CD-20), (ii) un plasmide (9 µg) d'expression de type CMV (promoteur du cytomegalovirus) sans séquence codante ou codant pour Mdm-2 (PBKCMVMdm2), pour le domaine 1-134 de Mdm-2 (PBKCMVMdm2(1-134)), E2F-4 ou E2F-5 (pCMVE2F-4, pCMVE2F-5), et (iii) un vecteur d'expression de p107 (pCMVp107, 9 µg). Les cellules sont ensuite traitées pour l'analyse par FACScan comme décrit par Zhu et al., Gene Dev., 1993, 7, 1111-1125). Les résultats d'une expérience représentative sont représentées dans la figure 4. Il démontre clairement que en l'absence de p107 surexprimé, l'expression de Mdm-2 ou de son domaine 1-134 n'ont pas d'effet sur le cycle cellulaire. En revanche, l'expression de Mdm-2 et, avec une efficacité, son domaine 1-134 sont capables de lever l'arrêt du cycle cellulaire en G1 induit par p107. Cet exemple démontre clairement que Mdm-2 n'est pas qu'un inhibiteur de l'activité transactivatrice de p53, mais aussi un régulateur positif du cycle cellulaire capable d'inhiber des facteurs impliqués dans le contrôle de celui-ci.

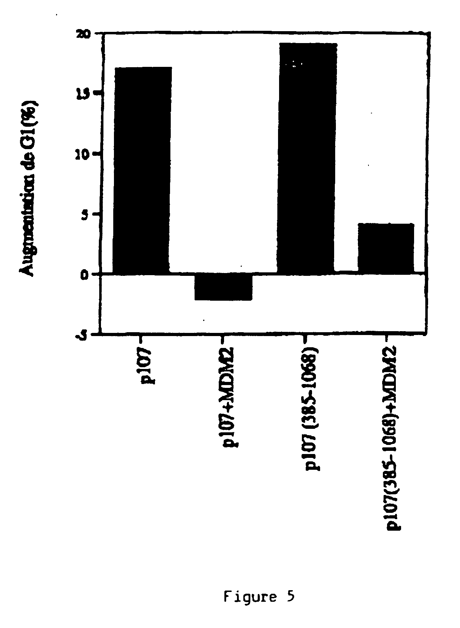

Dans une expérience similaire, des cellules Saos-2 sont co-transfectées avec 1 µg de p107(385-1068), 8 µg de pCMVNéoBam, 1µg de pXJMDM2, 8 µg de pXJ41 et 2 µg de pCMVCD20. Les résultats d'une expérience représentative sont indiqués sur la figure 5. Ils montrent que l'expression de MDM2 peut lever le blocage en G1 induit par p107 et par le mutant de délétion p107(385-1068) qui est capable d'interagir avec MDM2.

### Exemple 5: MDM2 interagit in vitro et in vivo avec p107

Cet exemple démontre une interaction physique entre MDM2 et p107, in vitro comme in vivo. Ces résultats sont corrélés avec l'activité de MDM2 au niveau du cycle cellulaire (exemple 4).

### 5.1. In vitro

p107 marqué au S35 in vitro est mis en contact avec de la protéine GST-MDM2 (vecteur pGex- MDM2) ou GST- MDM2(1-177) (vecteur pGex- MDM2(1-177)) immobilisée sur des billes glutathione sépharose. P107 liée à MDM2 est révélée après gel de polyacrylamide par autoradiographie. Les résultats obtenus sont présentés dans le Tableau II ci-après.

### 5.2. In vivo

Des cellules Cos sont cotransfectées avec un plasmide pBC- MDM2 ou pBC-MDM2(1-134) qui expriment une protéine de fusion GST- MDM2 ou GST-MDM2(1-134), avec un plasmide d'expression de p107 ou d'un mutant de p107. Les complexes de protéines GST- MDM2-p107 provenant des extraits cellulaires totaux sont isolés sur des billes glutathione sépharose et les protéines p107 sont révélées par Western blot avec un anticorps polyclonal anti-p107 (Santa Cruz p107-C18). Les résultats obtenus sont présentés dans le Tableau II ci-après.

| | p107 | p107(385-1068) | p107(1-781) | p107(385-1068) |
|---|---|---|---|---|
| **In Vivo** | | | | |
| GST- MDM2 | + | + | + | + |
| GST- MDM2(1-134) | + | nd | nd | nd |

| **In Vitro** | | | | |
|---|---|---|---|---|
| GST- MDM2 | + | nd | nd | nd |
| GST- MDM2(1-177) | + | nd | nd | nd |

Les résultats démontrent qu'il y a une interaction protéine-protéine entre MDM2 et p107 in vitro, mais aussi dans la cellule. La région de MDM2 nécessaire pour la transformation cellulaire (1-134) est la région qui interagit avec p107. Cette région a été localisée comme étant située plus précisément dans une partie du "pocket domain", région "A" et le "spacer".

## Revendications

1. Utilisation d'un composé capable d'antagoniser au moins partiellement l'activité oncogénique de la protéine Mdm2 pour la préparation d'une composition pharmaceutique destinée au traitement des cancers à contexte p53 nul.

2. Utilisation selon la revendication 1 d'un composé capable de se lier au niveau du domaine 1-134 de la séquence de la protéine Mdm2 représentée en SEQ ID N° 1.

3. Utilisation selon l'une des revendications 1 ou 2 **caractérisé en ce que** le composé est un scFV dirigé contre le domaine 1-134 de ladite protéine Mdm2.

4. Utilisation selon l'une des revendications 1 ou 2 **caractérisé en ce que** le composé est représenté en tout ou partie par un des peptides 1-52, 1-41, 6-41, 16-25, 18-23 de la séquence représentée en SEQ ID N°2 ou de leurs dérivés.

5. Utilisation selon la revendication 1 d'un composé capable de se lier à un domaine voisin du domaine 1-134 représenté en SEQ ID N° 1 de la protéine Mdm2 et affectant de part cette liaison l'activité oncogénique de ladite protéine.

6. Utilisation selon la revendication 1 ou 5 **caractérisé en ce que** le composé interagit avec le domaine C terminal de la protéine Mdm2.

7. Utilisation selon la revendication 1, 5 ou 6 **caractérisé en ce qu'**il s'agit d'un facteur transcriptionnel choisi parmi TFII, TBP et TAF250.

8. Utilisation selon la revendication 1 ou 5 **caractérisé en ce que** le composé interagit avec le domaine 135-491 de la protéine Mdm2.

9. Utilisation selon la revendication 1, 5 ou 8 **caractérisé en ce qu'**il s'agit en tout ou partie d'une protéine choisie parmi les protéines Rb, L5 et le facteur transcriptionnel E2F.

10. Utilisation d'un scFV dirigé contre le domaine 1-134 de la protéine Mdm2 pour la préparation d'une composition pharmaceutique destinée au traitement des cancers.

11. Utilisation d'un acide nucléique codant pour un composé capable d'antagoniser l'activité oncogénique de la protéine Mdm2 pour la préparation d'une composition pharmaceutique destinée au traitement des cancers à contexte p53 nul.

12. Utilisation selon la revendication 11 **caractérisée en ce qu'**il s'agit de :
- d'acides nucléiques antisens,
- d'oligonucléotides ligands capables de fixer directement l'un des domaines de la protéine Mdm2 et d'inhiber son activité oncogénique,
- d'acides nucléiques codant en tout ou partie pour des peptides ou protéines capables de s'oligomériser avec l'un des domaines de Mdm2 et d'inhiber son activité oncogénique,
- d'acides nucléiques codant pour des anticorps intracellulaires dirigés contre le domaine 1-134 de la séquence de la protéine Mdm2 représentée en SEQ ID N°1.

13. Utilisation selon la revendication 12 **caractérisé en ce que** l'acide nucléique antisens est un ADN codant pour un ARN complémentaire de l'acide nucléique codant pour la protéine Mdm2 et capable de bloquer sa transcription et/ou sa traduction (ARN antisens) ou un ribozyme.

14. Utilisation selon l'une des revendications 11 à 13 **caractérisée en ce que** l'acide nucléique est utilisé sous forme complexée avec du DEAE-dextran, avec des protéines nucléaires, ou avec des lipides ou polymères cationiques, sous forme de liposomes ou encore tel quel.

15. Utilisation selon l'une des revendications 11 à 13 **caractérisée en ce que** l'acide nucléique fait partie d'un vecteur.

16. Utilisation selon la revendication 15 **caractérisée en ce que** l'acide nucléique fait partie d'un vecteur viral, choisi parmi les adénovirus, les rétrovirus et les virus adéno-associés.

17. Vecteur viral carctérisé en ce que la séquence d'acides nucléiques code pour un scFv capable d'interagir au niveau du domaine 1-134 (SEQ ID N°1) de la protéine Mdm2.

18. Vecteur viral selon la revendication 18 **caractérisé en ce qu'**il est choisi parmi les adénovirus, les rétrovirus et les virus adéno-associés.

19. Vecteur viral selon les revendications 18 à 19 **caractérisé en ce qu'**il s'agit d'un adénovirus ou d'un rétrovirus.

20. Composition pharmaceutique comprenant un vecteur comprenant un acide nucléique codant pour un scFv capable d'interagir au niveau du domaine 1-134 (SEQ ID N°1) de la protéine Mdm2.

21. Composition pharmaceutique selon la revendication 20 **caractérisée en ce que** le vecteur est un vecteur plasmidique ou un vecteur viral choisi parmi les adénovirus, les rétrovirus ou les adéno-virus associés.

22. Composition selon la revendication 21 formulée en vue d'une administration intra-tumorale.

23. Utilisation d'une séquence nucléique codant pour des anticorps intracellulaires, dirigés contre le domaine 1-134 (SEQ ID N°1) de la protéine Mdm2 pour la préparation d'une composition pharmaceutique destinée au traitement du cancer.

## Patentansprüche

1. Verwendung einer Verbindung, die wenigstens teilweise die onkogene Aktivität des Proteins Mdm2 antagonisieren kann, zur Herstellung einer pharmazeutischen Zusammensetzung, die für die Behandlung der Krebserkrankungen ohne p53-Kontext bestimmt ist.

2. Verwendung gemäß Anspruch 1 einer Verbindung, die sich im Bereich der Domäne 1-134 der Sequenz des Proteins Mdm2, die in SEQ ID NO:1 dargestellt ist, binden kann.

3. Verwendung gemäß einem der Ansprüche 1 oder 2, **dadurch gekennzeichnet, dass** die Verbindung ein scFV ist, das gegen die Domäne 1-134 besagten Proteins Mdm2 gerichtet ist.

4. Verwendung gemäß einem der Ansprüche 1 oder 2, **dadurch gekennzeichnet, dass** die Verbindung ganz oder teilweise dargestellt wird durch eines der Peptide 1-52, 1-41, 6-41, 16-25, 18-23 der Sequenz, die in SEQ ID NO:2 dargestellt ist, oder von ihren Derivaten.

5. Verwendung gemäß Anspruch 1 einer Verbindung, die sich an eine benachbarte Domäne der Domäne 1-134, dargestellt in SEQ ID NO:1, des Proteins Mdm2 binden kann und durch diese Bindung die onkogene Aktivität besagten Proteins beeinflusst.

6. Verwendung gemäß Anspruch 1 oder 5, **dadurch gekennzeichnet, dass** die Verbindung mit der C-terminalen Domäne des Proteins Mdm2 interagiert.

7. Verwendung gemäß Anspruch 1, 5 oder 6, **dadurch gekennzeichnet, dass** es sich um einen Transkriptionsfaktor handelt, der ausgewählt ist unter TFII, TBP und TAF250.

8. Verwendung gemäß Anspruch 1 oder 5, **dadurch gekennzeichnet, dass** die Verbindung mit der Domäne 135-491 des Proteins Mdm2 interagiert.

9. Verwendung gemäß Anspruch 1, 5 oder 8, **dadurch gekennzeichnet, dass** es sich ganz oder teilweise um ein Protein handelt, das ausgewählt ist unter den Proteinen Rb, L5 und dem Transkriptionsfaktor E2F.

10. Verwendung eines scFV, das gegen die Domäne 1-134 des Proteins Mdm2 gerichtet ist, für die Herstellung einer pharmazeutischen Zusammensetzung, die für die Behandlung der Krebserkrankungen bestimmt ist.

11. Verwendung einer Nukleinsäure, die für eine Verbindung kodiert, die die onkogene Aktivität des Proteins Mdm2 antagonisieren kann, zur Herstellung einer pharmazeutischen Zusammensetzung, die für die Behandlung der Krebserkrankungen ohne p53-Kontext bestimmt ist.

12. Verwendung gemäß Anspruch 11, **dadurch gekennzeichnet, dass** es sich handelt um:
- Antisense-Nukleinsäuren,
- Oligonukleotidliganden, die eine der Domänen des Proteins Mdm2 direkt fixieren und seine onkogene Aktivität inhibieren können,
- Nukleinsäuren, die ganz oder teilweise für Peptide oder Proteine kodieren, die sich mit einer der Domänen von Mdm2 oligomerisieren und seine onkogene Aktivität inhibieren können,
- Nukleinsäuren, die für intrazelluläre Antikörper kodieren, die gegen die Domäne 1-134 der Sequenz des Proteins Mdm2, dargestellt in SEQ ID NO: 1, gerichtet sind.

13. Verwendung gemäß Anspruch 12, **dadurch gekennzeichnet, dass** die Antisense-Nukleinsäure eine DNA ist, die für eine komplementäre RNA der Nukleinsäure, die für das Protein Mdm2 kodiert und seine Transkription und/oder seine Translation (Antisense-RNA) blockieren kann, oder ein Ribozym kodiert.

14. Verwendung gemäß einem der Ansprüche 11 bis 13, **dadurch gekennzeichnet, dass** die Nukleinsäure in komplexierter Form mit DEAE-Dextran, mit Zellkernproteinen oder mit Lipiden oder kationischen Polymeren, in Form von Liposomen oder auch so, wie sie ist, verwendet wird.

15. Verwendung gemäß einem der Ansprüche 11 bis 13, **dadurch gekennzeichnet, dass** die Nukleinsäure Teil eines Vektors ist.

16. Verwendung gemäß Anspruch 15, **dadurch gekennzeichnet, dass** die Nukleinsäure Teil eines viralen Vektors ist, der ausgewählt ist unter den Adenoviren, den Retroviren und den adeno-assoziierten Viren.

17. Viraler Vektor, **dadurch gekennzeichnet, dass** die Nukleinsäurensequenz für ein scFv kodiert, das im Bereich der Domäne 1-134 (SEQ ID NO:1) des Proteins Mdm2 interagieren kann.

18. Viraler Vektor gemäß Anspruch 18, **dadurch gekennzeichnet, dass** er ausgewählt ist unter den Adenoviren, den Retroviren und den adeno-assoziierten Viren.

19. Viraler Vektor gemäß den Ansprüchen 18 bis 19, **dadurch gekennzeichnet, dass** es sich um ein Adenovirus oder ein Retrovirus handelt.

20. Pharmazeutische Zusammensetzung, umfassend einen Vektor, umfassend eine Nukleinsäure, die für ein scFv kodiert, das im Bereich der Domäne 1-134 (SEQ ID NO:1) des Proteins Mdm2 interagieren kann.

21. Pharmazeutische Zusammensetzung gemäß Anspruch 20, **dadurch gekennzeichnet, dass** der Vektor ein Plasmidvektor oder ein viraler Vektor ist, der ausgewählt ist unter den Adenoviren, den Retroviren oder den adeno-assoziierten Viren.

22. Zusammensetzung gemäß Anspruch 21, formuliert in Hinblick auf eine intratumorale Verabreichung.

23. Verwendung einer Nukleinsequenz, die für intrazelluläre Antikörper kodiert, die gegen die Domäne 1-134 (SEQ-ID NO:1) des Proteins Mdm2 gerichtet sind, für die Herstellung einer pharmazeutischen Zusammensetzung, die für die Behandlung von Krebs bestimmt ist.

## Claims

1. Use of a compound capable of antagonizing, at least partially, the oncogenic activity of the Mdm2 protein for the preparation of a pharmaceutical composition intended for the treatment of cancers with a zero p53 context.

2. Use according to Claim 1 of a compound capable of binding at the level of the 1-134 domain of the sequence of the Mdm2 protein represented in SEQ ID No. 1.

3. Use according to either of Claims 1 and 2, **characterized in that** the compound is an scFV directed against the 1-134 domain of the said Mdm2 protein.

4. Use according to either of Claims 1 and 2, **characterized in that** the compound is represented, completely or in part, by one of the peptides 1-52, 1-41, 6-41, 16-25, 18-23 of the sequence represented in SEQ ID No. 2 or of their derivatives.

5. Use according to Claim 1 of a compound capable of binding to a domain close to the 1-134 domain represented in SEQ ID No. 1 of the Mdm2 protein and affecting, by virtue of this binding, the oncogenic activity of the said protein.

6. Use according to Claim 1 or 5, **characterized in that** the compound interacts with the C-terminal domain of the Mdm2 protein.

7. Use according to Claim 1, 5 or 6, **characterized in that** it involves a transcriptional factor chosen from TFII, TBP and TAF250.

8. Use according to Claim 1 or 5, **characterized in that** the compound interacts with the 135-491 domain of the Mdm2 protein.

9. Use according to Claim 1, 5 or 8, **characterized in that** it involves, completely or in part, a protein chosen from the proteins Rb, L5 and the transcriptional factor E2F.

10. Use of an scFV directed against the 2-134 domain of the Mdm2 protein for the preparation of a pharmaceutical composition intended for the treatment of cancers.

11. Use of a nucleic acid encoding a compound capable of antagonizing the oncogenic activity of the Mdm2 protein for the preparation of a pharmaceutical composition intended for the treatment of cancers with a zero p53 context.

12. Use according to Claim 11, **characterized in that** it involves:
- antisense nucleic acids,
- ligand oligonucleotides capable of directly binding one of the domains of the Mdm2 protein and of inhibiting its oncogenic activity,
- nucleic acids encoding, completely or in part, peptides or proteins capable of oligomerizing with one of the domains of Mdm2 and of inhibiting its oncogenic activity,
- nucleic acids encoding intracellular antibodies directed against the 1-134 domain of the sequence of the Mdm2 protein represented in SEQ ID No. 1.

13. Use according to Claim 12, **characterized in that** the antisense nucleic acid is a DNA encoding an RNA complementary to the nucleic acid encoding the Mdm2 protein and capable of blocking its transcription and/or its translation (antisense RNA) or a ribozyme.

14. Use according to one of Claims 11 to 13, **characterized in that** the nucleic acid is used in a form complexed with DEAE-dextran, with nuclear proteins, or with cationic polymers or lipids, in the form of liposomes or alternatively as it is.

15. Use according to one of Claims 11 to 13, **characterized in that** the nucleic acid forms part of a vector.

16. Use according to Claim 15, **characterized in that** the nucleic acid forms part of a viral vector, chosen from adenoviruses, retroviruses and adeno-associated viruses.

17. Viral vector **characterized in that** the nucleic acid sequence encodes an scFv capable of interacting at the level of the 1-134 domain (SEQ ID No. 1) of the Mdm2 protein.

18. Viral vector according to Claim 17, **characterized in that** it is chosen from adenoviruses, retroviruses and adeno-associated viruses.

19. Viral vector according to Claims 17 and 18, **characterized in that** it is an adenovirus or a retrovirus.

20. Pharmaceutical composition comprising a vector comprising a nucleic acid encoding an ScFv capable of interacting at the level of the 1-134 domain (SEQ ID No. 1) of the Mdm2 protein.

21. Pharmaceutical composition according to Claim 20, **characterized in that** the vector is a plasmid vector or a viral vector chosen from adenoviruses, retroviruses or adeno-associated viruses.

22. Composition according to Claim 21, formulated for intratumoral administration.

23. Use of a nucleic sequence encoding intracellular antibodies, directed against the 1-134 domain (SEQ ID No. 1) of the Mdm2 protein for the preparation of a pharmaceutical composition intended for the treatment of cancer.
